(19) Europäisches Patentamt
European Patent Office
Office européen des brevets

(11) Veröffentlichungsnummer: **0 004 621**
**B1**

# (12) EUROPÄISCHE PATENTSCHRIFT

(45) Veröffentlichungstag der Patentschrift: **09.05.84**

(21) Anmeldenummer: **79100901.2**

(22) Anmeldetag: **24.03.79**

(51) Int. Cl.³: **C 07 C 175/00,** C 07 D 317/72, C 07 C 49/703, C 07 C 35/08 // C07C69/16, C07C69/145, C07C79/20, C07F9/54

## (54) Verfahren zur mehrstufigen Synthese von Zeaxanthin und Alloxanthin über Cyclohexanon- und Cyclohexanolderivate; Cyclohexanon- und Cyclohexanolderivate.

(30) Priorität: **27.03.78 US 890173**

(43) Veröffentlichungstag der Anmeldung: **17.10.79 Patentblatt 79/21**

(45) Bekanntmachung des Hinweises auf die Patenterteilung: **09.05.84 Patentblatt 84/19**

(84) Benannte Vertragsstaaten: **BE CH DE FR GB IT NL**

(56) Entgegenhaltungen:
**FR - A - 1 151 878**
**FR - A - 2 272 971**
**FR - A - 2 357 541**
**GB - A - 1 173 063**

(73) Patentinhaber: **F. HOFFMANN-LA ROCHE & CO. Aktiengesellschaft, CH-4002 Basel (CH)**

(72) Erfinder: **Saucy, Gabriel, 125 Fells Road, Essex Fells, N.J. (US)**

(74) Vertreter: **Aschert, Hubert et al, Postfach 601 Grenzacherstrasse 124, CH-4002 Basel (CH)**

EP 0 004 621 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die vorliegende Erfindung betrifft ein Verfahren zur Herstellung von Cyclohexanderivaten, welche als Zwischenprodukte zur stereospezifischen Synthese von Zeaxanthin und Alloxanthin, natürlichen Lebensmittelfarbstoffen, ausgehend von 2,6,6-Trimethyl-1,4-cyclohexandion geeignet sind.

Die Erfindung betrifft ferner einige dieser Zwischenprodukte selbst und die Verwendung einiger dieser Zwischenprodukte zur Herstellung von Alloxanthin bzw. Zeaxanthin.

Die oben genannte stereospezifische Synthese führt zu den natürlichen Lebensmittelfarbstoffen der Formeln

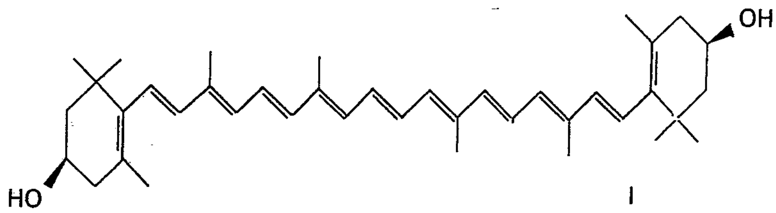

I

und

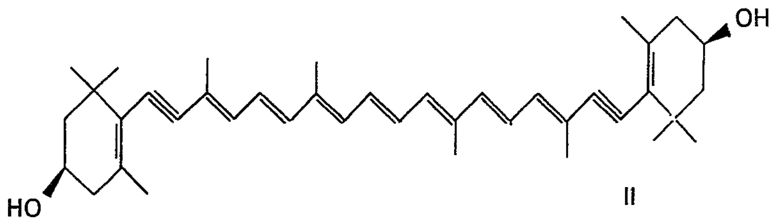

II

und

ausgehend von der Verbindung der Formel

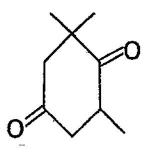

III

Aus GB-A-1173063 und FR-A-1151878 sind Synthesen für Carotinoide bzw. Vorstufen davon bekannt, welche es jedoch nicht ermöglichen, die Carotinoide in der gewünschten stereospezifischen Form zu erhalten.

Gemäss einer Ausführungsform des erfindungsgemässen Verfahrens wird die Verbindung der Formel III stereospezifisch in ein Zwischenprodukt der Formel

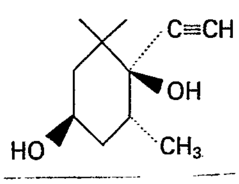

IV

übergeführt.

Die Verbindung der Formel IV stellt, aufgrund ihrer Stereokonfiguration, ein Zwischenprodukt für die Herstellung von Zeaxanthin (Verbindung der Formel I) dar. Aufgrund der Stereokonfiguration der Verbindung der Formel IV und aufgrund des erfindungsgemässen Verfahrens ist es möglich, natürliches Zeaxanthin herzustellen, in welchem die Hydroxygruppen an jedem der beiden Cyclohexenringe die β-Konfiguration aufweisen.

In den hier dargestellten Formeln sind die verschiedenen Substituenten mit den Ringkomponenten durch verschiedene Bindungen verbunden, nämlich: einen Keil (◀), welcher anzeigt, dass der betreffende Substituent β-orientiert ist (d. h. oberhalb der Papierebene liegt) und eine unterbrochene Linie (---), welche anzeigt, dass der betreffende Substituent α-orientiert ist (unterhalb der Papierebene liegt).

Der Ausdruck «nieder Alkyl» bezieht sich auf gesättigte aliphatische Kohlenwasserstoffgruppen, welche 1-7 Kohlenstoffatome enthalten, beispielsweise Methyl, Aethyl, n-Propyl, Isopropyl, Isobutyl und dergleichen. Der Ausdruck «Halogen» schliesst alle vier Halogenatome ein, also Chlor, Fluor, Jod und Brom. Der Ausdruck «Alkalimetall» bezieht sich auf Lithium, Natrium, Kalium, Rubidium und Cäsium.

Der Ausdruck «nieder Alkanoyl» bezieht sich auf niedere Alkanoylsubstituenten, welche 2-7 Kohlenstoffatome enthalten, beispielsweise Acetyl und Butyryl. Der Ausdruck «Aryl» schliesst mononukleare Arylgruppen, beispielsweise Phenyl, welche an einer oder mehreren Stellen mit niederen Alkylgruppen substituiert sein können, sowie polynukleare Arylgruppen, wie Naphthyl, Anthryl, Phenanthryl und Azalyl ein, welche ebenfalls mit einer oder mehreren niederen Alkylgruppen substituiert sein können. Der Ausdruck «Aroyl» bezieht sich auf Aroylgruppen, worin der Arylanteil die obige Bedeutung hat. Die bevorzugte Aroylgruppe ist die Benzoylgruppe.

Gemäss der vorliegenden Erfindung wird die Verbindung der Formel III über die folgenden Zwischenprodukte in die Verbindung der Formel IV übergeführt:

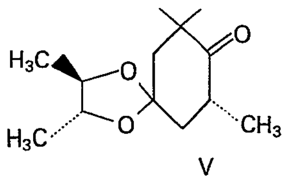

V

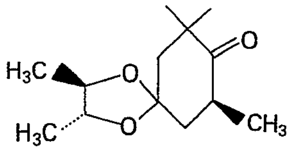

V-A

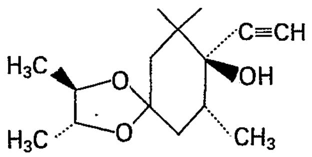

VI

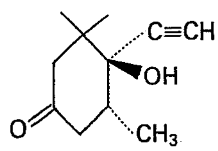

VII

Zwecks Überführung der Verbindung der Formel III in die Verbindung der Formel V wird die Verbindung der Formel III mit D(-)-Butylenglykol der Formel

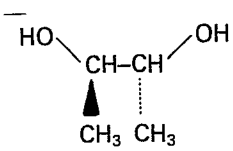

X

umgesetzt. Die Umsetzung der Verbindung der Formel III mit der Verbindung der Formel X kann nach einer für die Ketalisierung eine Ketons mit einem Alkylenglykol üblichen Methode durchgeführt werden. Bevorzugt wird die Umsetzung der Verbindung der Formel III mit der Verbindung der Formel X in Gegenwart eines sauren Katalysators durchgeführt. Als saure Katalysatoren können hierbei beispielsweise starke anorganische Säuren, wie Schwefelsäure, und starke organische Säuren, beispielsweise p-Toluolsulfonsäure, verwendet werden. Im allgemeinen wird diese Umsetzung in einem inerten organischen Lösungsmittel, beispielsweise in einem aromatischen Kohlenwasserstoff, wie Benzol, Toluol und dergleichen, durchgeführt. Zweckmässig führt man die Umsetzung bei der Rückflusstemperatur des Reaktionsgemisches durch, wobei man das gebildete Wasser azeotrop entfernt.

Bei der Umsetzung der Verbindung der Formel III mit der Verbindung der Formel X erhält man die Verbindung der Formel V im Gemisch mit der Verbindung der Formel V-A. Aus diesem Gemisch kann die Verbindung der Formel V leicht entfernt werden, da diese in inerten organischen Lösungsmitteln weniger löslich ist als die Verbindung der Formel V-A und daher aus dem Gemisch leicht durch Kristallisation abgetrennt werden kann. Nach Umsetzung der Verbindung der Formel III mit der Verbindung der Formel X kann daher die Verbindung der Formel V aus dem Reaktionsgemisch auskristallisiert werden, wobei die Verbindung der Formel V-A, gegebenenfalls zusammen mit geringen Mengen der Verbindung der Formel V, in der Mutterlauge zurückbleibt.

Die Verbindung der Formel V-A kann in die Verbindung der Formel V übergeführt werden, und zwar entweder direkt in der Mutterlauge oder nach Isolierung aus der Mutterlauge. Vorzugsweise wird die Überführung der Verbindung der Formel V-A in die Verbindung der Formel V ohne vorherige Isolierung der Verbindung der Formel V-A durchgeführt. Diese Überführung erfolgt mittels Aequilibrierung, wobei man vorzugsweise so vorgeht, dass man die Verbindung der Formel V-A oder Gemische, welche diese Verbindung enthalten, mit einer starken Base in Gegenwart eines inerten organischen Lösungsmittels behandelt. Als starke Basen können übliche Basen verwendet werden, beispeilsweise Alkalimetallhydroxyde und Erdalkalimetallhydroxyde, beispielweise Natriumhydroxyd, Calciumhyroxyd, Kaliumhydroxyd usw. Ferner können als starke Basen Alkalimetallcarbonate, beispielsweise Natriumcarbonat oder Kaliumcarbonat verwendet werden. Auch Alkalimetall-nieder-alkoxyde, wie Natriummethoxyd, können als starke Basen eingesetzt werden. Die Aequilibrierung erfolgt zweckmässig in einem inerten organischen Lösungsmittel, beispielsweise Diäthyläther, Dioxan, niederen Aklanolen, wie Methanol oder Aethanol, aliphatischen Kohlenwasserstoffen, wie Hexan. Erwünschtenfalls kann auch Wasser im Reaktionsmedium vorhanden sein. Die angewandten Temperaturen und Drucke sind nicht kritisch; es kann beipielsweise bei Raumtemperatur und Atmosphärendruck gearbeitet werden, oder auch bei Temperaturen zwischen 10°C und 150°C.

Die Verbindung der Formel V wird in die Verbindung der Formel VI übergeführt, und zwar kann dies durch Umsetzung der Verbindung der Formel V mit einer Verbindung der Formel

X–Mg–C=CH XI

worin X Hologen bedeutet, erfolgen.

Diese Umsetzung wird unter den üblichen Bedingungen einer Grignard-Synthese durchgeführt. Die Überführung der Verbindung der Formel V in die Verbindung der Formel VI kann aber auch durch Umsetzung der Verbindung der Formel V mit einem Alkalimetallacetylid, beispielsweise Natriumacetylid oder Lithiumacetylid erfolgen, wobei man unter den üblichen, für die Reaktion eines Ketons mit einem Acetylid geeigneten Bedingungen arbeitet. Üblicherweise wird diese Umsetzung in flüssigem Ammoniak durchgeführt.

Die Verbindung der Formel VI wird durch saure Hxdrolyse in die Verbindung der Formel VII übergeführt; dies kann in üblicher, für die Überführung eines Ketals in das entsprechende Keton geeigneter Weise geschehen. Zweckmässig kann diese Hydrolyse mittels einer organischen Säure, beispielsweise Essigsäure, bei Temperaturen zwischen etwa 50 und 100°C erfolgen.

Die Verbindung VII wird durch Hydrierung mit einem nieder Alkoxy-alkalimetall-aluminiumhydrid in die Verbindung der Formel IV übergeführt. Bevorzugte derartige Hydirde sind das Lithium (tritert.-butoxy)-aluminiumhydrid und das Natriumbis-(2-methoxy-äthoxy)-aluminiumhydrid. Vorzugsweise wird diese Reduktion in einem inerten organischen Lösungsmittel, beispielsweise Aethylenchlorid, Benzol oder Toluol durchgeführt. Temperatur und Druck sind hierbei nicht kritisch. Man kann bei Temperaturen zwischen etwa 10 und 100°C arbeiten, beispielsweise bei Raumtemperatur und Atmosphärendruck.

Die Verbindung der Formel IV kann nach der im folgenden Beispiel 5 beschriebenen Weise in Zeaxanthin übergeführt werden.

In einer weiteren Ausführungsform kann das erfindungsgemässe Syntheseprinzip für die Herstellung von Alloxanthin (Verbindung der Formel II), und zwar über die folgenden Zwischenprodukte:

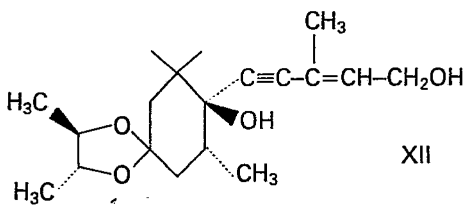
XII

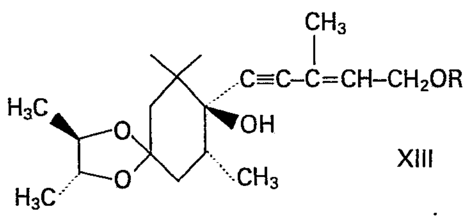
XIII

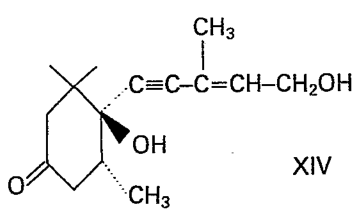
XIV

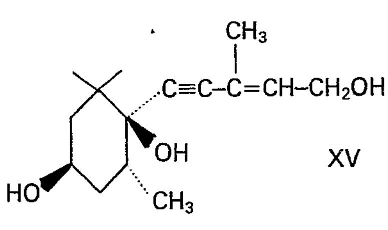
XV

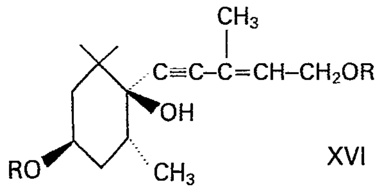
XVI

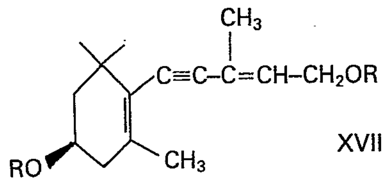
XVII

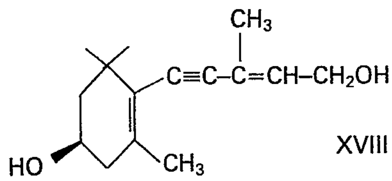
XVIII

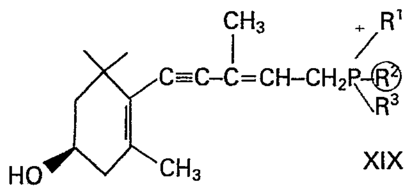
XIX

worin R niederes Alkanoyloxy oder Aroyloxy und $R^1$, $R^2$ und $R^3$ niederes Alkyl oder Aryl bedeuten, und X die obige Bedeutung hat.

Zwecks Überführung der Verbindung der Formel V in die Verbindung der Formel XII wird die Verbindung der Formel V mit einem Organometallhalogenid der Formel

$$\text{X–MgC}\equiv\text{C–}\overset{\overset{\displaystyle CH_3}{|}}{\text{C}}\text{=CH–CH}_2\text{OH} \qquad \text{XX}$$

5 4 3 2 1

worin X die obige Bedeutung hat, umgesetzt.

In der Verbindung der obigen Formel XX können die Substiuenten an der 2,3-Doppelbindung in trans- oder in cis-Konfiguration stehen oder es kann die Verbindung der Formel XX in Form von cis-trans-Gemischen vorliegen. Die geometrische Konfiguration im Endprodukt (Alloxanthin) richtet sich nach der geometrischen Konfiguration im Zwischenprodukt der Formel XX. Wenn daher ein trans-Endprodukt erwünscht ist, so ist als Seitenkettekomponente auch eine trans-Verbindung einzusetzen.

Die Umsetzung der Verbindung der Formel XX mit der Verbindung der Formel V erfolgt in für Grignard-Synthesen üblicher Weise, wobei dieselben Bedingungen angewandt werden können, wie

sie bei der Umsetzung der Verbindung der Formel V mit der Verbindung der Formel XI zur Verbindung der Formel VI beschrieben wurden. Man erhält hierbei die Verbindung der Formel XII im Gemisch mit einer Verbindung der Formel

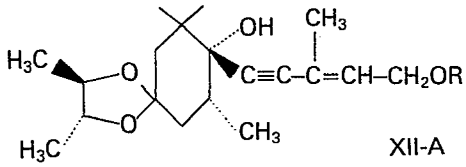

XII-A

Das so erhaltene Gemisch der Formel XII und XII-A kann in üblicher Weise getrennt werden. Beispielsweise kann die Trennung dadurch erfolgen, dass man die Verbindung der Formel XII verestert, d. h. in eine Verbindung der Formel XIII überführt. Dieser Veresterung kann in üblicher Weise mit einem reaktionsfähigen Derivat einer niederen Alkancarbonsäure oder einer Arylcarbonsäure durchgeführt werden. Bevorzugt wird die Verbindung der Formel XII in den Benzoesäureester übergeführt. Dies kann beispielsweise erfolgen durch Umsetzung mit einem Benzoesäurehalogenid. Die im Gemisch nach der Veresterung vorliegenden Ester können in einfacher Weiser, beispielsweise mittels Chromatographie, getrennt werden. Nach der Abtrennung der Verbindung der Formel XIII wird diese mit einer Base hydrolysiert, wobei man die Verbindung der Formel XII in reiner Form erhält.

Die Überführung der Verbindung der Formel XII in die Verbindung der Formel XIV erfolgt durch Ketalspaltung, in ähnlicher Weise wie sie im Zusammenhang mit der Überführung der Verbindung der Formel VI in die Verbindung der Formel VII beschrieben wurde. Die Verbindung der Formel XIV wird hierauf zur Verbindung der Formel XV reduziert, und zwar durch Behandlung mit einem Alkalimetallnieder-alkoxy-aluminiumhydrid. Diese Reaktion kann in derselben Weise erfolgen, wie sie in Verbindung mit der Überführung der Verbindung der Formel VII in die Verbindung der Formel IV beschrieben wurde. Die Verbindung der Formel XV wird in die Verbindung der Formel XIV durch Veresterung mit einem reaktionsfähigen Derivat einer niederen Alkancarbonsäure oder Arylcarbonsäure übergeführt. Diese Veresterung kann in üblicher Weise erfolgen.

Die Verbindung der Formel XVI wird durch Behandlung mit einem Dehydratisierungsmittel in die Verbindung der Formel XVII übergeführt. Bevorzugte Dehydratisierungsmittel sind saure Dehydratisierungsmittel, beispielsweise Phosphoroxychorid oder Schwefelsäure. Im allgemeinen wird bei Temperaturen zwischen etwa 60 und 120°C gearbeitet, wobei Rückflusstemperatur bevorzugt ist. Zweckmässig wird die Umsetzung in einem inerten organischen Lösungsmittel durchgeführt.

Die erhaltene Verbindung der Formel XVIII kann mittels einer Ylidreaktion mit einem Hydrohalogenid eines Phosphins der Formel

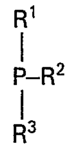

XXI

worin $R_1$, $R_2$ und $R_3$ die obige Bedeutung haben, in die Verbindung der Formel XIX übergeführt werden.

Die Verbindung der Formel XVIII wird hierbei mit mit dem Hydrohalogenidsalz der Verbindung der Formel XXI zweckmässig in Gegenwart eines inerten organischen Lösungsmittels umgesetzt. Als Hydrohalogenidsalz ist das Triphenylphosphin-hydrobromid bevorzugt. Als Lösungsmittel werden halogenierte Kohlenwasserstoffe, wie Dichlormethan, bevorzugt. Druck und Temperatur sind nicht kritisch; die Umsetzung kann bei Raumtemperatur und atmosphärischem Druck durchgeführt werden.

Die Verbindung der Formel II erhält man durch Umsetzung der Verbindung der Formel XIX mit einer Verbindung der Formel

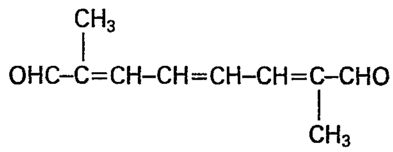

XXII

mittels einer Wittig-Reaktion. Diese Reaktion wird unter den für Wittig-Reaktionen üblichen Bedingungen durchgeführt. Es werden pro Mol der Verbindung der Formel XXII zwei Mol der Verbindung der Formel XIX verwendet.

Während in der obigen Beschreibung die zwei Ausführungformen, nämlich die Herstellung von Zeaxanthin einerseits und von Allixanthin andererseits, getrennt beschrieben wurden, können diese beiden Ausführungsformen auch durch ein einziges Formelschema dargestellte werden, worin Z Wasserstoff (im Falle der Herstellung von Zeaxanthin), bzw. die Gruppe

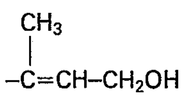

IIa

(im Falle der Herstellung von Alloxanthin) bedeutet.

Dieses einheitliche Formelschema ist im folgenden dargestellt, und zwar für diejenigen Teile der beiden Ausführungsformen, welche die Reaktionsfolgen III→IV (für Zeaxanthin) und III→XV (für Alloxanthin) umfassen:

Reaktionsschema

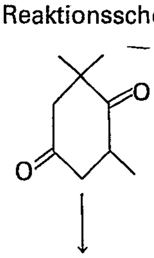

III

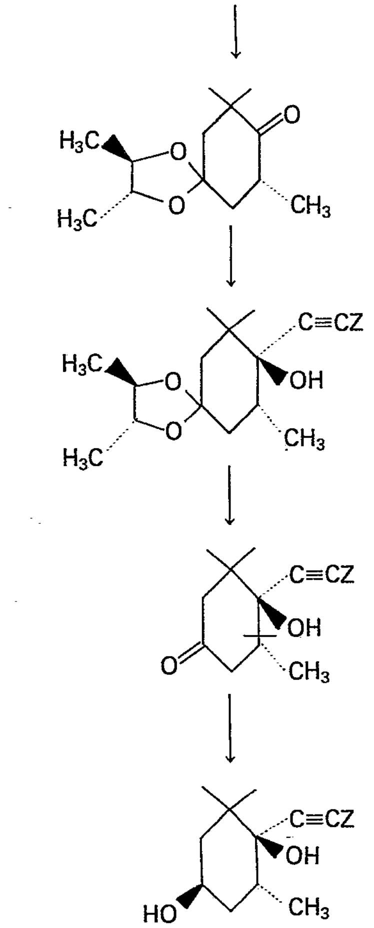

V

VI,XII

VII,XIV

IV,XV

100 g 2,2,6-Trimethyl-1,4-cyclohexandion, 77 g (-)-2,3-Butandiol, 2 g p-Toluolsulfonsäure und 1000 ml Benzol werden in einen Reaktionskolben mit Wasserabscheider eingebracht und während 4 Stunden am Rückfluss erhitzt. Das Reaktionsgemisch wird auf Raumtemperatur abgekühlt und dann in 250 ml gesättigte, wässerige Natriumbicarbonatlösung gegossen. Die organische Phase wird abgetrennt, gewaschen mit Salzwasser, getrocknet über Magnesiumsulfat und das Lösungsmittel unter vermindertem Druck abgedampft. 152 g des erhaltenen Rohmaterials werden gelöst in einer Mischung von 100 ml Tetrachlorkohlenstoff und Hexan (1:1 Volumenanteile) und langsam, zuerst bei Raumtemperatur und dann in einem Kühlschrank bei −15°C während 2 Wochen, auskristallisiert. Aus der Mutterlauge werden 21,5 g 2(R),3(R),7,7,9(R)-Pentamethyl-1,4-dioxaspiro[4,5]-decan-8-on in Form grosser Kristalle erhalten und über Nacht unter vermindertem Druck getrocknet. Nach dem Umkristallisieren aus Tetrachlorkohlenstoff/Hexan beträgt der Schmelzpunkt des Produktes 71-72°C.

127,4 g der Mutterlauge, die sich durch Säulenchromatographie (Carbowax) als eine Mischung von 2(R),3(R),7,7,9(S)-Pentamethyl-1,4-dioxaspiro[4,5]-decan-8-on im Gewichtsverhältnis 1:1,5 herausstellite, werden gelöst in 500 ml einer 5-Gew.%igen, wässerigen Natriumhydroxydlösung in 95 vol.%igem, wässrigem Methanol und während 2 Tagen auf 60°C erhitzt. Der Grossteil des Lösungsmittels (über 300 ml) wird abgedampft unter vermindertem Druck. Der Rückstand wird gelöst in Äther, fünfmal mit Wasser gewaschen, getrocknet über Magnesiumsulfat und das Lösungsmittel abgedampft bei vermindertem Druck. Aus 105,7 g Rohmaterial werden 10,2 g 2(R),3(R),7,7,9(R)-Pentamethyl-1,4-dioxaspiro[4,5]-decan-8-on auskristallisiert.

Zusätzlich 11,3 g 2(R),3(R),7,7,9(R)-Pentamethyl-1,4-dioxaspiro[4,5]-decan-8-on werden erhalten aus 81,1 g Rohmaterial nach Behandlung der Mutterlauge (86,4 g) mit 5 vol.%iger, methanolischer Natriumhydroxydlösung. Das entspricht gesamthaft 29,2% der theoretischen Ausbeute. Die letzte Mutterlauge (68,4 g) enthält immer noch mehr als 75% des Isomerengemisches von 2(R),3(R),7,7,9(R)-Pentamethyl-1,4-dioxaspiro[4,5]-decan-8-on und 2(R),3(R),7,7,9(S)-Pentamethyl-1,4-dioxaspiro[4,5]decan-8-on.

Acetylen-Gas wird durch ein Rohr in einen 500 ml Dreihalskolben mit einer Grignard-Lösung eingeleitet. Diese wird hergestellt aus 1,56 g Magnesiumspäne und 8,16 g Aethylbromid in 100 ml getrocknetem Tetrahydrofuran bei 10-15°C (Eis-Wasser-Bad) während 2 Stunden und unter Argon. Dem Reaktionsgemisch werden zusätzlich 80 ml Tetrahydrofuran zugegeben, um zu vermeiden, dass das Grignard-Salz ausfällt.

6,78 g 2(R),3(R),7,7,9(R)-Pentamethyl-1,4-dioxaspiro[4,5]-decan-8-on in 70 ml Tetrahydrofuran werden bei 10°C tropfenweise zugegeben und dann während 17 Stunden die erhaltene Mischung unter Argon bei Raumtemperatur gerührt. Das Reaktionsgemisch wird in 100 ml gesättigte, wässrige Ammoniumchloridlösung gegossen und die organische Phase abgetrennt. Diese wird vereinigt mit zwei 100 ml-Aetherextrakten der wässrigen Phase, gewaschen mit Salzwasser und getrocknet über Magnesiumsulfat. Nach Abdampfen der Lösungsmittel unter vermindertem Druck werden 7,8 g (Ausbeute ca. 100%) Rohprodukt von 8(S)-Aethinyl-2(R),3(R),7,7,9(R)-pentamethyl-1,4-dioxaspiro[4,5]-decan-8-ol erhalten. Kristallisation aus Isopropyläther ergibt 6,3 g (83,2%) 8(S)-Aethinyl-2(R),3(R),7,7,9(R)-pentamethyl-1,4-dioxaspirol[4,5]-decan-8-ol (Schmelzpunkt 79-83°C; GC Analyse: > 98% Reinheit). Eine analytische Probe wird erhalten durch Umkristallisation aus Isopropyläther: Schmelzpunkt 83-85°C.

5 g(S)-Aethinyl-2(R),3(R),7,7,9(R)-pentamethyl-1,4-dioxaspiro[4,5]-decan-8-ol (Schmelzpunkt 79-83°C), die nach Beispiel 3 erhalten wurden, werden in 30 ml einer 80 vol.-%igen, wässrigen Essigsäurelösung aufgelöst und während 3 Stunden bei 70°C gerührt. Die Lösung wird mit Natriumchlorid gesättigt und mehrmals mit Methylenchlorid extrahiert. Die organischen Extrakte werden vereinigt, nacheinander gewaschen mit Salzwasser und schliessliche über Magnesiumsulfat getrocknet. Nach Entfernen des Lösungsmittels unter vermindertem Druck werden 3,6 g (ca. 100%) des Rohproduktes 4(S),5(R)-4-Aethinyl-4-hydroxy.-3,3,5-trimethylcyclohexan-1-on erhalten (97% nach GC-Analyse). Kristallisation aus Aether ergibt 3,09 g (85,8%) Substanz mit einem

Schmelzpunkt von 140-143°C. Eine analytische Probe wird durch Umkristallisation aus Aether--.Hexan erhalten; Schmelzpunkt 141–143°C.

1,2g des kristallinen 4(S),5(R)-4-Aethinyl-4-hydroxy-3,3,5-trimethylcyclohexan-1-ons werden in 20ml getrocknetem Tetrahydrofuran gelöst und dann langsam, bei 0-5°C, einer Suspension von 3,2g Lithium-tri-tert.-butoxy-aluminiumhydrid in 40ml getrocknetem Tetrahydrofuran zugegeben. Die Mischung wird gerührt, zuerst während 30 Minuten bei 5°C, dann 17 Stunden bei Raumtemperatur und schliesslich 1 Stunde lang am Rückfluss leicht erhitzt. Bei 0°C werden unter Rühren (pH ca. 6) sorgfältig 16ml einer 5 vol.-%igen, wässrigen Essigsäurelösung hinzugegeben. Die Mischung wird mit Natriumchlorid gesättigt und mehrmals mit Aether extrahiert. Nach dem Trocknen der organischen Phase über Magnesiumsulfat und Entfernen der Lösungsmittel unter vermindertem Druck werden 1,53g Rohmaterial erhalten. Kristallisation aus Aether-Hexan liefert 0,900g (74,1%) der Verbindung 1(S),4(R),6(R)-1-Aethinyl-2,2,6-trimethyl-1,4-cyclohexandiol (Schmelzpunkt 150-152°C; GC-Analyse: Reinheit > 98%). Umkristallisation dieser Substanz ergibt 0,850g Reinprodukt, Schmelzpunkt 150-152°C; GC: keine Verunreinigung; $[\alpha]^{25}_{D} = -24,93$ (1% in Dioxan).

In einem alternativen Reduktionsverfahren werden 0,540g 4(S),5(R)-4-Aethinyl-4-hydroxy-3,3,5-trimethylcyclohexan-1-on in Benzol tropfenweise zu 2,1g Bis(2-methoxyäthoxy)-aluminiumhydrid (70% in Benzol) gegeben, was 0,518g des gewünschten Isomeres 1(S),4(R)6(R)-1-Aethinyl-2,2,6-trimethyl-1,4-cyclohexandiol (nach GC-Analyse) liefert. Die Reduktionsbedingungen sind gleich wie oben.

0,140g reines 1(S),4(R),6(R)-1-Aethinyl-2,2,6-trimethyl-1,4-cyclohexandiol werden in 2ml einer Acetanhydrid-Pyridin-Mischung (1:1 Volumenteile) gelöst und 17 Stunden bei Raumtemperatur unter Argon gerührt. Die entstehende hellgelbe Lösung wird bei 70°C/12mm Hg bis zur Trockenheit eingeengt. Dabei werden 0,156g Rohprodukt 1(R),4(S),5(R)-4-Aethinyl-4-hydroxy-3,3,5-trimethyl cyclohexylacetat erhalten; Schmelzpunkt 69,5-72,5°C; $[a]^{25}_{D} = -22,85$ (1% in Dioxan). Umkristallisation aus Aether-Hexan liefert 0,129g reines 1(R),4(S),5(R)-4-Aethinyl-4-hydroxy-3,3,5-trimethyl-cyclohexylacetat. Zu 33,3g dieser Verbindung werden, unter Argon und unter dauerndem Rühren, 360g Mesitylen, 0,395 Stearinsäure, 8,28g Triphenylsilanol und 1,76g Triisopropylorthovanadol zugegeben. Die entstandene Mischung wird dann bei 140°C unter Argon 22 Stunden gerührt. Die Mischung, die ursprünglich hellgelb ist, wird allmählich dunkelbraun. Das Reaktionsgemisch wird auf 40°C gekühlt und dann destilliert, zuerst unter Wasserstrahlvakuum und nachher unter Hochvakuum. Der dunkelbraune, ölige Rückstand wird mit 175ml Petroläther gemischt und filtriert. Nach Entfernen des Petroläthers wird der ölige Rückstand auf einer Silicagel-Säule (Teilchengrösse 0,03 bis 0,2mm) chromatographiert. Die Säule wird mit n-Hexan aufbereitet und die Elution mit n-Hexan/Diäthyläther 1:1 ausgeführt. Auf diese Weise werden 30,0g reines (1R)-4-(Formylmethyl)-3,5,5-trimethyl-3-cyclohexen-1-ylacetat erhalten; Ausbeute 90%, $[\alpha]^{25}_{D} = -110°$ (c = 1 in Dioxan).

Zu einer Mischung von 28,8g des obigen Produktes und 395ml Nitroäthan wird unter Rühren und bei 22°C unter Argon-Atmosphäre 1ml einr 40%igen, methanolischen Lösung von Benzyl-trimethyl-ammoniumhydroxyd gegeben. Nach 40 Stunden bei 25°C wird die Lösung verdünnt mit 1700ml Diäthyläther. Die organische Phase wird gewaschen mit 2N Schwefelsäure (3 × 100ml) und dann mit gesättigter, wässriger Natriumchloridlösung (5 × 100ml) neutral gewaschen. Nach der Trocknung über Natriumsulfat und Destillation unter vermindertem Druck bei 40°C werden 38,4g (1R)-4-(2-Hydroxy-3-nitrobutyl)-3,3,5-trimethyl-3-cyclohexen-1-ylacetat als Öl erhalten.

Zu einem Gemisch von 27,6g des oben erhaltenen Öls und 110ml Acetanhydrid bei 25°C werden unter Rühren 0,4ml frisch destilliertes Bortrifluorid-Aetherat gegeben. Die Mischung wird 16 Stunden bei 25°C stehengelassen und dann über 1 Liter Eiswasser gegossen. Während 30 Minuten wird weitergerührt und dann das Produkt mit Methylenchlorid extrahiert. Die Methylenchlorid-Extrakte werden zuerst mit gesättigter, wässriger Natriumchloridlösung und 1N Schwefelsäure gewaschen und dann mit verdünnter, wässriger Natriumcarbonatlösung neutral gewaschen. Durch Trocknung über Natriumsulfat und Destillaion des Produktes bei 40°C unter reduziertem Druck werden 31,5g (1R)-4-(2-Acetoxy-3-nitrobutyl)-3,5,5-trimethyl-3-cyclohexen-1-ylacetat (Ausbeute 100%) erhalten. 15,8g dieses Produktes werden in 160ml Benzol gelöst und dann 31,5g Aluminiumoxyd zugegeben. Diese Mischung wird während 22 Stunden auf 80°C erwärmt und gerührt unter Argon. Anschliessend wird das Reaktionsgemisch auf Raumtemperatur abgekühlt und nach Zugabe von 1,5g Aktivkohle das Produkt filtriert und bei 40°C unter vermindertem Druck destilliert. Der Rückstand wird dann aus einer warmen Diäthyläther/n-Hexan-Mischung umkristallisiert und ergibt 8,85g (1R)-3,5,5-Trimethyl-4-(3-nitro-2-butenyl)-3-cyclohexen-1-ylacetat (Ausbeute 69%). Schmelzpunkt 61-62°C; $[\alpha]^{25}_{D} = -79,3°$ (c = 1 in Dioxan).

Zu einer Lösung von 18,1g des im vorangehenden Abschnitt erhaltenen Produktes in 181ml Tetrahydrofuran werden 19,5g Triäthylamin hinzugefügt und dann wird die Mischung 5 Tage bei 25°C gerührt. Das Produkt wird anschliessend auf 3°C abgekühlt und mit 10%iger, wässriger Schwefelsäure versetzt, bis ein pH-Wert von 3,5 erreicht ist. Während 10 Minuten wird weitergerührt, dann 900ml Eiswasser zugegeben und die Mischung mit Diäthyläther extrahiert. Die vereinigten Aether-Extrakte werden mit gesättigter Natriumchloridlösung gewaschen und dann mit Wasser neutral gewaschen. Das Produkt wird getrocknet und destilliert und liefert 16,0g Rohprodukt (3R)-3-Acetoxy.β-ionon (Ausbeute 99%). Dieses wird chromatographisch gereinigt auf 480Silicagel (Teilchengrösse 0,06 bis 0,2mm). Die Säule wird mit n-Hexan aufbereitet und die Elution, mit n-Hexan/

Diäthyläther (1:1) ausgeführt, ergibt 6,6 Reinprodukt als dunkelgelbes, viskoses, schwach nach Veilchen riechendes Oel (Ausbeute 39,4%).

6,0g reines (3R)-3-Acetoxy-β-ionon werden in 60ml Methanol gelöst, mit 6,0ml 22,4%iger (Gew./Vol.) wässriger Kaliumhydroxydlösung gemischt und die erhaltene Lösung 2 Stunden bei 25°C gerührt. Das Reaktionsprodukt wird dann mit 90g Eis gemischt, mit Natriumchlorid gesättigt und extrahiert mit Diäthyläther. Die vereinigten Äther-Extrakte werden mit kalter, gesättigter, wässriger Natriumchloridlösung neutral gewaschen, getrocknet über Natriumsulfat und destilliert. Auf diese Art werden 4,75g Rohprodukt (3R)-3-Hydroxy-β-ionon oder 4-[(4R)-4-Hydroxy-2,6,6-trimethyl-1-cyclohexen-1-yl]-3-butenon (Ausbeute 95%) erhalten, das dann chromatographiert auf 165g Silicagel (Teilchengrösse 0,06 bis 0,2mm) mit Diäthyläther als Eluierungsmittel, 4,1g (3R)-3-Hydroxy-β-ionon (Ausbeute 80%) liefert. Das (3R)-3-Hydroxy-β.-ionon wird in all-trans [3R,3'R]-Zeaxanthin umgewandelt gemäss Beispiel 13 der Deutschen Offenlegungsschrift (DOS) 2 537 060.

Zu einer Grignard-Lösung von 1,55g Magnesium, aktiviert mit Jod, und 7,00g Aethylbromid in 100ml getrocknetem Tetrahydropyran werden bei 10-15°C (kaltes Wasserbad) tropfenweise 2,6g Transpentol (1-Hyroxy-3-methyl-2-penten-4-in) in 25ml getrocknetem Tetrahydrofuran zugegeben. Das Reaktionsgemisch wird während 30 Minuten bei Raumtemperatur gerührt und dann 20 Minuten auf 50°C erwärmt. Das Wasserbad wird entfernt, und bei Raumtemperatur werden tropfenweise 5,5g 2(R),3(R),7,7,9(R)Pentamethyl-1,4-dioxaspiro[4,5]-decan-8-on (erhalten nach Beispiel 1), gelöst in 25ml getrocknetem Tetrahydrofuran, zugegeben. Das Reaktionsgemisch wird in 50ml gesättigte Ammoniumchloridlösung gegossen und der Reaktionskolben mit Äther ausgespült. Die Ätherlösung wird zweimal mit Salzwasser gewaschen und über Magnesiumsulfat getrocknet. Durch Eindampfen unter vermindertem Druck werden 9,1g Substanz erhalten. Dieses Rohprodukt wird auf Aluminiumoxyd (364g) chromatographiert. Äther-Hexan 4:1 (Volumenteile) eluiert ein Gemisch von 5,67g (72% der Theorie) 8(S)-E-(5-Hydroxy-3-methylpent-3-en-1-inyl)-2(R), 3(R),7,7, 9(R)-pentamethyl-1,4-dioxaspiro[4,5]-decan-8-ol und 8(R)-E-(5-Hydroxy-3-methylpent-3-en-l-inyl)-2(R),3(R),7,7,9(R)-pentamethyl-1,4-dioxaspiro[4,5]-decan-8-ol.

6,1g des Isomerengemisches 8(S)-E-(5-Hydroxy-3-methylpent-3-en-1-inyl)-2(R),3(R),7,7,9(R)-pentamethyl-1,4-dioxaspiro[4,5]-decan-8-ol und 8(R)-E-(5-Hydroxy-3-methylpent-3-en-1-inyl)-2(R),3(R),7,7,9(R)-pentamethyl-1,4-dioxaspiro[4,5]-decan-8-ol, gelöst in 60ml Pyridin, werden bei Raumtemperatur mit 3,7g Benzolchlorid behandelt. Das Reaktionsgemisch wird bei Raumtemperatur 17 Stunden gerührt, anschliessend verdünnt mit Äther und die organische Phase gewaschen, zweimal mit gekühlter 1N Schwefelsäure und je einmal mit Salzwasser, gesättigter Natriumcarbonalösung und Salzwasser. Nach der Trocknung über Magnesiumsulfat und Entfernung der Lösungsmittel bleiben 8,4g (ca. 100%) Rohprodukt, die auf etwa 210g Aluminiumoxyd chromatographiert werden. Benzol-Äther (9:1 Volumenteil) eluiert 5,31g 8(S)-E-(5-Benzoyloxy-3-methylpent-3-en-1-inyl)-2(R),3(R),7,7,9(R)-pentamethyl-1,4-dioxaspiro[4,5]-decan-8-ol.

4,53g 8(S)-E-(5-Benzoyloxy-3-methylpent-3-en-l-inyl)-2(R),3(R),7,7,9(R)-pentamethyl-1,4-dioxaspiro[4,5]-decan-8-ol werden behandelt mit 40ml einer Lösung von 5 Gew.-% Kaliumhydroxyd in 95%igem wässrigem Methanol. Die Mischung wird während 30 Minuten am Rückfluss erhitzt, und dann werden unter vermindertem Druck 20ml Methanol abgedampft. Der Rest wird mit Aether verdünnt und dreimal mit Salzwasser gewaschen. Die organische Phase wird über Magnesiumsulfat getrocknet, und nach Abdampfen des Lösungsmittels unter vermindertem Druck werden 3,6g Rohpodukt 8(S)-E-(5-Hydorxy-3-methylpent-3-en-1-inyl)-2(R),3(R),7,7,9(R)-pentamethyl-1,4-dioxaspiro[4,5]-decan-8-ol (ca. 100%) erhalten. Die Kristallisation aus Hexan-Aether liefert 3,25g Substanz, die für die Verwendung in der nächsten Stufe rein genug ist (Schmelzpunkt 85-87°C). Eine analytische Probe, die durch mehrmalige Kirstallisation aus Hexan-Aether erhalten wird, schmilzt bei 86,5-87,5°C.

1g 8(S)-E-(5-Hydroxy-3-methylpent-3-en-1-inyl)-2(R),3(R),7,7,9(R)-pentamethyl-1,4-dioxaspiro[4,5]-decan-8-ol in 30ml Aceton wird mit 2ml 1N wässriger Schwefelsäure behandelt und bei 50°C 24 Stunden gerührt. Die Säure wird mit 1N wässriger Natriumhydroxydlösung neutralisiert und dann unter reduziertem Druck das Aceton grösstenteils entfernt. Der Rest (10ml Lösung) wird mit Äther verdünnt, die organische Phase zweimal mit Salzwasser gewaschen, über Magnesiumsulfat getrocknet und unter vermindertem Druck eingedampft. Dabei werden 0,820g 1(S)-E-(5-Hydroxy-3-methyl-3-penten-1-inyl)-2,2,6(R)-trimethyl-cyclohexan-4-on-1-ol (90,5% nach GC-Analyse) erhalten.

Zu einer Suspension von 3g Tri-tert.-butoxyaluminiumhydrid in 15ml Tetrahydrofuran werden bei Raumtemperatur 0,82g 1(S)-E-(5-Hydroxy-3-methyl-3-penten-1-inyl)-2,2,6(R)-trimethylcyclohexan-4-on-l-ol, gelöst in 5ml Tetrahydrofuran, gegeben und diese Mischung über Nacht bei gleicher Temperatur gerührt. Der Überschuss des Reagenses wird wird mit 5%iger, wässriger Essigsäure (pH ca. 5) zerstört. Der Kolben wird mit Äther ausgespült, die Ätherextraktionen werden zweimal mit Salzwasser gewaschen und dann über Magnesiumsulfat getrocknet. Nach dem Eindampfen bei vermindertem Druck werden 0,805g Rohprodukt erhalten. Die Kristallisation aus Äther ergibt 0,545g (ca. 70% der Theorie) reines 1(S)-E-(5-Hydroxy-3-methyl-3-penten-1-inyl)-2,2,6(R)-trimethyl-1,4(R)-cyclohexandiol. Eine analytische Probe, die durch mehrmalige Kristallisation aus Methanol-Äther erhalten wurde, schmilzt bei 128-130,5°C.

50mg 1(S)-E-(5-Hydroxy-3-methyl-3-penten-1-inyl)-2,2,6(R)-trimethyl-1,4(R)-cyclohexandiol, gelöst in 1ml einer Acetanhydrid-Pyridin-Mischung

(1:1 Volumenteile), werden über Nacht bei Raumtemperatur gerührt. Nach Abdampfen der Lösungsmittel bleiben 62 mg Rohprodukt, das aus Äther-Hexan umkristallisiert 60 mg reines 4(R)-Acetoxy-1(S)-E-(5-acetoxy-3-methyl-3-penten-1-inyl)-2,2,6(R)-trimethyl-1-cyclohexanol (Schmelzpunkt 86-87°C; $[\alpha]^{25}_{D}$ = 21,59, 1% in Dioxan; GC-Analyse: Reinheit 97%; NMR: keine Isomere nachgewiesen) ergibt.

20,16 g dieser Verbindung, gelöst in 250 ml Pyridin, werden zuerst bei Raumtemperatur und dann 17 Stunden bei 95-100°C, mit 40 ml Phosphoroxychlorid (d = 1,67) behandelt. Das Reaktionsgemisch wird portionenweise in einem grossen Erlenmeyer-Kolben eingebracht, in welchem sich zerkleinertes Eis und Wasser befindet, und dann mit 110 g festem Natriumbicarbonat neutralisiert. Das Produkt wird mit Äther extrahiert. Die Ätherlösung wird dreimal mit Wasser gewaschen, über wasserfreiem Magnesiumsulfat getrocknet, und dann werden unter vermindertem Druck die Lösungsmittel entfernt. 12,9 g Rohprodukt werden auf Aluminiumoxyd (Qualität III) chromatographiert. Eine Mischung von Hexan-Benzol 4:1 eluiert 9,8 g (51,5%) des Diacetat-Produktes, welches dann mit 50 ml einer Lösung von 5% Kaluimhydroxyd in 95%igem Methanol behandelt wird. Nach 30 Minuten Rühren bei Raumtemperatur wird der Grossteil des Lösungsmittels bei vermindertem Druck verdampft. Der Rückstand wird mit Äther extrahiert, mit Salzwasser neutral gewaschen und über wasserfreiem Magnesiumsulfat getrocknet.

8 g des Rohproduktes, das nach Entfernen des Lösungsmittels erhalten wird, ergeben mit Tetrahydrofuran/Diäthyläther umkristallisiert 4,2 g (58,2%) reines E-5-Hydroxy-[4(R)-hydroxy-2,6,6-trimethyl-1-cyclohexen-1-yl]-3-methyl-3-penten-1-in. Schmelzpunkt 94-98°C.

Eine Mischung von 2,34 g dieser Verbindung und 3,85 g Triphenylphosphoniumhydrobromid, gelöst in 30 ml Methylenchlorid, wird bei Raumtemperatur 17 Stunden gerührt. ⅔ des Gesamtvolumens des Reaktionsgemisches werden unter vermindertem Druck abgedampft und dann Aethylacetat in die übriggebliebene, konzentrierte Lösung getropft, bis diese trübe wird. Wenn die Mutterlauge einen Tag bei −15°C stehengelassen wird, scheiden sich 3,5 g kristalline Substanz aus. Das erhaltene (E)-[5-(4R-Hydroxy-2,6,6-trimethyl-1-cyclohexen-1-yl)-3-methyl-2-penten-4-inyl]-triphenylphosphoniumbromid schmilzt bei 184-186°C.

Zu einer Suspension von 2,795 g dieses Phosphoniumsalzes in 20 ml Methylenchlorid werden bei Raumtemperatur und unter Argon 0,328 g trans-2,7-Dimethylocta-2,4,6-trien-1,8-dial gegeben. Während die Mischung gerührt wird, werden in etwa 20 Minuten 0,145 g Natriummetall, gelöst in 2 ml Methanol, zugegeben. Nach 2 Stunden Rühren bei 30°C wird das Reaktionsgemisch in eine Natriumcarbonatlösung gegossen und daraus das Produkt mit Methylenchlorid extrahiert. Nach Entfernen des Lösungsmittels werden 2,9 g eines dunkelroten Öles erhalten. Dieses wird in einer kleinen Menge eines Methylenchlorid-Methanol-Gemisches (1:1) gelöst und auf 5°C abgekühlt. Nach Filtration, Waschen mit Äther/Methanol und Trocknen unter vermindertem Druck werden 0,425 g kristalline Substanz erhalten, die nach mehrmaliger Kristallisation, reines all-trans-Alloxanthin (Schmelzpunkt 178,5-180,5°C) ergibt.

**Patentansprüche**

1. Verfahren zur Herstellung von Cyclohexanderivaten, dadurch gekennzeichnet, dass man eine Verbindung der Formel

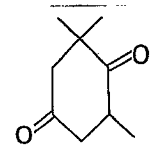

III

mit einem Glykol der Formel

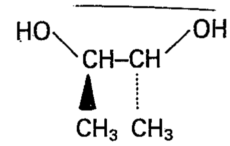

X

zu einem Ketal der Formel

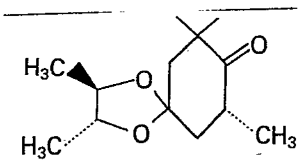

V

umsetzt, dass man das erhaltene Ketal zu einer Verbindung der Formel

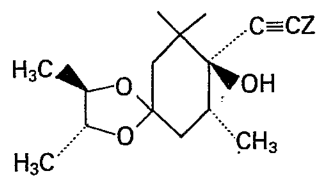

VI,XII

alkinyliert, worin Z Wasserstoff oder die Gruppe

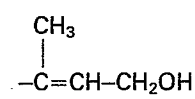

II a

bedeutet, dass man die erhaltene Alkinverbindung zu dem entsprechenden Keton de Formel

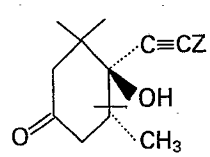

VII,XIV

hydrolysiert und dass man das erhaltene Keton zur entprechenden Hydroxyverbindung der Formel

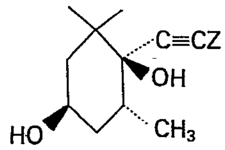

IV,XV

hydriert.

2. Verfahren nach Anspruch 1, dadurch gekennzeichnet, dass die Alkinylierung des Ketals der Formel V durch Umsetzung mit einer Verbindung der Formel

X-Mg-C≡CZ XI,XX

erfolgt, worin X Halogen und Z Wasserstoff oder die Gruppe

$CH_3$
|
$-C{=}CH{-}CH_2OH$ II a

bedeutet.

3. Ein Verfahren nach Anspruch 1 oder 2, dadurch gekennzeichnet, dass man die Reduktion des Ketons der Formel VII, XIV mittels eines nieder Alkoxy-alkalimetallaluminiumhydrids durchführt.

4. Eine Verbindung der Formel

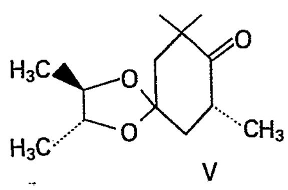

V

nämlich 2 (R),3(R),7,7,9(R)-Pentamethyl-1,4-dioxaspiro[4,5]-decan-8-on.

5. Verbindungen der Formel

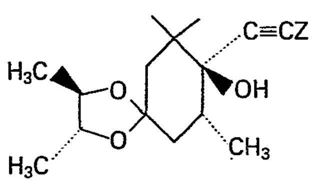

VI,XII

worin Z Wasserstoff oder die Gruppe

$CH_3$
|
$-C{=}CH{-}CH_2OH$ II a

bedeutet.

6. Eine Verbindung nach Anspruch 5, nämlich 8(S)-Aethinyl-2-(R),3(R),7,7,9(R)-pentamethyl-dioxaspiro[4,5]-decan-8-ol.

7. Eine Verbindung nach Anspruch 5, nämlich 8(S)-E-(5 Hydroxy-3-methyl-3-penten-1-inyl)-2(R), 3(R),7,7,9(R)-pentamethyl-1,4-dioxaspiro[4,5] decan-8-ol.

8. Verwendung einer nach dem Verfahren nach einem der Ansprüche 1-3 erhaltenen Verbindung der Formel IV, XV zur Herstellung von Alloxyanthin oder Zeaxanthin durch Dehydratisierung und Ylidreaktion.

**Claims**

1. Process for the manufacture of cyclohexane derivatives, characterized by reacting a compound of the formula

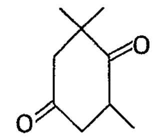

III

with a glycol of the formula

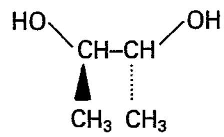

X

to give a ketal of the formula

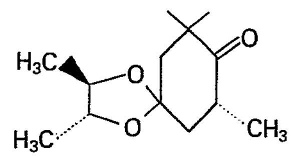

V

alkynylating the resulting ketal to give a compound of the formula

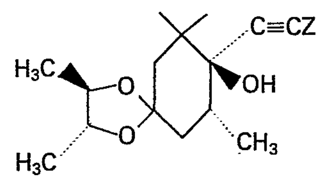

VI,XII

wherein Z signifies hydrogen or the group

$CH_3$
|
$-C{=}CH{-}CH_2OH$ II a

hyrolyzing the resulting alkyne compound to give the corresponding ketone of the formula

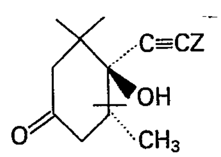

VII,XIV

and hydrogenating the resulting ketone to give the corresponding hydroxy compound of the formula

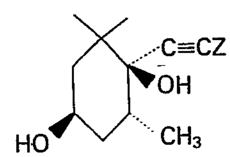

IV,XV

2. Process according to claim 1, characterized in that the alkynylation of the ketal of formula V is carried out by reaction with a compund of the formula

X-Mg-C≡CZ XI, XX

wherein X signifies halogen and Z signifies hydrogen or the group

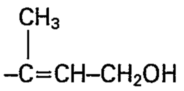

II a

3. A process according to claim 1 or 2, characteriszed in that the reduction of the ketone of formula VII, XIV is carried out by means of a lower alkoxy-alkali metal aluminium hydride.

4. A compounds of the formula

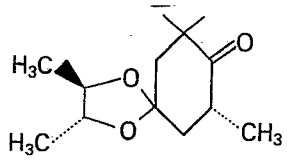

V

namely 2(R),3(R),7,7,9(R)-pentamethyl-1,4-dioxaspiro[4,5]-decan-8-one.

5. Compounds of the formula

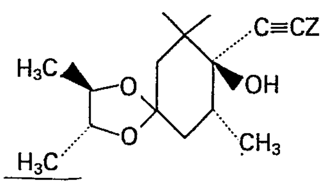

VI,XII

wherein Z signifies hydrogen or the group

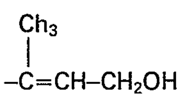

II a

6. A compound according to claim 5, namely 8 (S)-ethynyl-2-(R),3(R),7,7,9(R)-pentamethyl-dioxaspiro[4,5]-decan-8-ol.

7. A compound according to claim 5, namely 8(S)-E-(5-hydroxy-3-methyl-3-penten-1-ynyl)-2(R),3(R),7,7,9(R)-pentamethyl-1,4-dioxaspiro[4,5]decan-8-ol.

8. Use of a compound of formula IV, XV obtained according to the process according to any one of claims 1-3 for the manufacture of alloxanthin or zeaxanthin by dehydration and ylid reaction.

**Revendication**

1. Procédé de préparation de dérivés du cyclohexane, caractérisé en ce que, en faisant réagir un composé de formule:

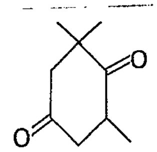

III

avec un glycol de formule:

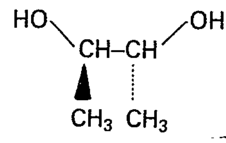

X

on forme un cétal de formule:

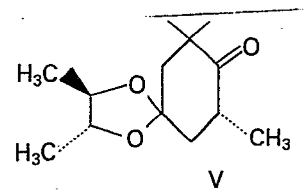

V

qu'on soumet à alcynylation, ce qui donne un composé de formule:

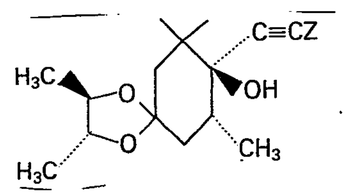

VI,XII

dans laquelle Z représente l'hydrogène ou le groupe:

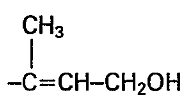

II a

on hydrolyse le composé alcynylique obtenu en la cétone correspondante de formule:

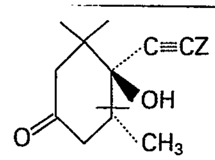

VII,XIV

qu'on hydrogène en le composé hydroxylé correspondant de formule:

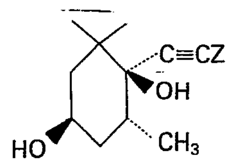

IV,XV

2. Procédé selon la revendication 1, caractérisé en ce que l'alcynylation de le cétal de formule V est réalisée par réaction avec un composé de formule:

X–Mg–≡CZ XI, XX

dans laquelle X représente un halogène et Z représente l'hydrogène ou le groupe

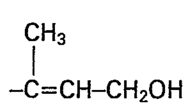

II a

3. Un procédé selon la revendication 1 ou 2, caractérisé en ce que la réduction de la céotone de formule VII, XIV est effectuée à l'aide d'un hydrure

d'(alcoxy inférieur)-métal alcalin-aluminium.

4. Un composé de formule:

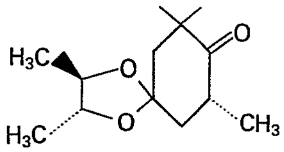

V

à savoir la 2(R),3(R),7,7,9(R)-pentaméthyl-1,4-dioxaspiro[4,5]-décane-8-one.

5. Composés de formule:

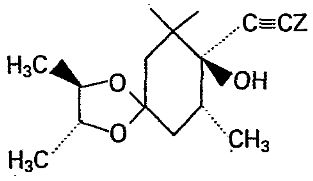

VI,XII

dans laquelle Z représente l'hydrogène ou le groupe

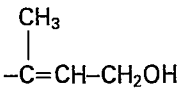

6. Un composé selon la revendication 5, à savoir le 8(S)-éthenyl-2(R),3(R),7,7,9(R)-pentaméthyl-dioxaspiro[4,5]-décane-8-ol.

7. Un composé selon la revendication 5, à savoir le 8(S)-E-(5-hydroxy-3-méthyl-2-pentène-1-ynyl)-2(R),3(R),7,7,9(R)-pentaméthyl-1,4-dioxaspiro[4,5]-décane-8-ol.

8. Utilisation d'un composé de formule IV, XV obtenu par le procédé selon l'une des revendications 1 à 3, pour la préparation de l'alloxanthine ou de la zéaxanthine par déshydratation et réaction de formation d'ylide.